# EUROPEAN PATENT APPLICATION

(11) **EP 1 521 192 A1**
(43) Date of publication of application: **06.04.2005**
(21) Application number: 03762858.3
(22) Date of filing: 06.03.2003
(51) Int. Cl.: G06F 17/60, G06F 17/50

(54) **METHOD AND APPARATUS OF ANALYSING SUGAR CHAIN STEROSTRUCTURE**

(30) Priority: 05.07.2002 JP 2002197639
(71) Applicant: FUJITSU LIMITED, Kawasaki-shi, Kanagawa 211-8588 (JP)
(72) Inventor: IKEDA, Noriko, Fujitsu Limited, Kawasaki-shi, Kanagawa 211-8588 (JP)
(74) Representative: Sunderland, James Harry
(86) International application number: PCT/JP2003/002674
(87) International publication number: WO 2004/006149

(57) **Abstract**

The present invention provides a three-dimensional structure analysis method that extracts monosaccharide bonding patterns significant for elucidation of the formation of the three-dimensional structures of polysaccharides when the alignments of local structures are given for those polysaccharides. This three-dimensional structure analysis method comprises inputting the alignment of the local structures of a polysaccharide; inputting coordinate data relating to polysaccharides to which each local structure belongs; detecting interactions between substituents of each hydroxyl group within a local structure and hydroxyl groups that serve as acceptors of other monosaccharides; evaluating the interaction patterns; and outputting information relating to the interactions.

## Description

### TECHNICAL FIELD

The present invention relates to polysaccharide analysis technology, and more particularly, to a method and device for analyzing the three-dimensional structure of polysaccharides in support of the elucidation of the mechanism of polysaccharide three-dimensional structure formation by detecting a pattern of interaction with a characteristic environment in which those local structures are commonly observed or by detecting a polysaccharide having a similar pattern of interaction based on the alignment of the local structures of polysaccharides. In addition, the present invention relates to a computer program and recording medium that are used to carry out this polysaccharide analysis method and device. Moreover, the present invention relates to a diagnostic support method, and more particularly, to a method for providing support for the diagnosis of diseases and so forth by detecting a feature observed in common in the three-dimensional structures of polysaccharides based on polysaccharide three-dimensional structural information obtained in accordance with the present invention.

### BACKGROUND ART

As is well-known, polysaccharides (sugar chains) are monosaccharides such as glucose, galactose or mannose that have been bonded in the form of chains (hereinafter, referred to as "monosaccharide linkages"), and are present by bonding to proteins and so forth on the surfaces of cells. The functions of polysaccharides cover a wide range due to their diversity, and have been clearly demonstrated to be intimately involved in various biological phenomena such as development, differentiation, growth, reproduction, aging and carcinogenesis. The functions demonstrated by polysaccharides depend on their three-dimensional structure and the physicochemical properties associated with their three-dimensional structure. In many cases, however, it is extremely difficult to determine their three-dimensional structure directly from genome information since the structure is modified following translation. Consequently, there is a need to develop a method for predicting the three-dimensional structure of polysaccharides from their monosaccharide bonding in order to develop vaccines and other pharmaceuticals, design new functional materials, elucidate the complex physiological functions of the body and resolve a wide range of other unanswered questions.

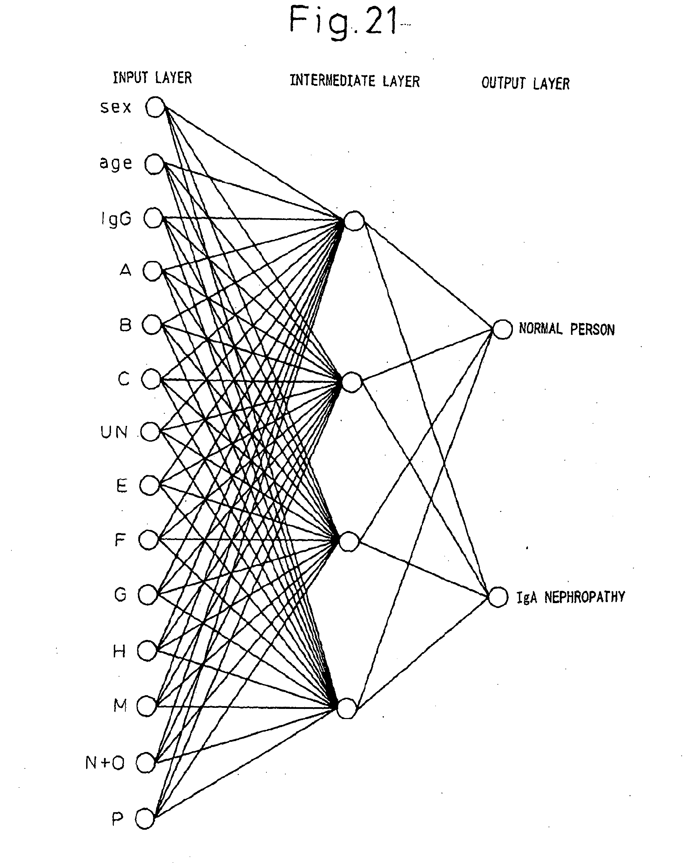

In addition, since polysaccharides are intimately involved in biological phenomena as previously described, in the case of having measured the content of a plurality of polysaccharides, it is expected to be possible to provide support for diagnosing diseases and so forth based on that polysaccharide content. For example, in order to achieve this object, Japanese Unexamined Patent Publication (Kokai) No. 9-257790 proposes a method for supporting diagnosis of diseases and so forth based on clinical data. In this diagnostic support method, data correlating to the concentrations of a plurality of polysaccharides sampled from the body is treated with a neural network to obtain diagnostic support results. According to this diagnostic support method, support for diagnosing IgA nephropathy, for example, can be provided by using a neural network for identification of IgA nephropathy patients shown in Fig. 21.

In looking at the diagnostic support technology of the prior art proposed in the aforementioned unexamined patent publications and other documents, diagnostic support is provided by investigating the symptoms of a disease. However, changes in the structures and amounts of proteins caused by abnormalities in homeostasis responsible for causing a disease cannot be extracted by this technology of the prior art. One reason for this is presumed to be that considerations are only given to dealing with symptoms. Thus, in the symptom-oriented technology of the prior art as described above, since features of polysaccharides were not taken into consideration, there is the serious problem of being unable to obtain enough data for elucidating principles and developing methods for supporting diagnoses. In addition, since the HPLC method is used to detect polysaccharides in the diagnostic support method of Japanese Unexamined Patent Publication (Kokai) No. 9-257790, there is also the problem of quantitative measurements being difficult.

### DISCLOSURE OF THE INVENTION

Although it can be said to be difficult to determine the three-dimensional structure of a polysaccharide directly from genome information or experimentally, ever since the three-dimensional structures of proteins have been determined experimentally, the three-dimensional structures of several hundred polysaccharides for which sample preparation is difficult have been reported in the protein databank (PDB). In addition, observation of their three-dimensional structures reveals that there are local structural patterns that appear in common with several polysaccharides.

Therefore, an object of the present invention is to construct empirical rules for correlating monosaccharide bonding with polysaccharide three-dimensional structure and use these rules to predict three-dimensional structure by extracting monosaccharide bonding patterns characteristic of these known local structural patterns, and extracting polysaccharides having similar interaction patterns.

In looking at related extraction technologies of the prior art with respect to proteins, the extraction of amino acid sequence patterns characteristic of structural patterns has been carried out by preparing alignments of several local structures having that structural pattern, and then investigating whether or not a peculiar amino acid pattern is present for each location of the alignment. However, significant monosaccharide bonding patterns cannot be extracted depending on the technology of the prior art. One of the reasons for this is presumed to be that only amino acid sequences within local structures were taken into consideration when monosaccharide bonding patterns are extracted using the aforementioned method. Accordingly, the inventors of the present invention found that, since the interaction of hydroxyl group substituents greatly contributes to the formation of the three-dimensional structure of polysaccharides in terms of monosaccharide bonding, it is necessary to take into consideration whether or not the interaction between substituents of hydroxyl groups that form local structures and their surrounding environments, and hydroxyl groups serving as acceptors of other monosaccharides bonded with α- or β-glucoside bonds by C1 hydroxyl groups involved in bonding between monosaccharides, is characteristic. In addition, the inventors of the present invention also found as a result of additional research that it is also necessary to incorporate the features of existing patterns and take into consideration polysaccharides having similar interaction patterns by focusing on the three-dimensional structures of polysaccharides along with the physicochemical properties of those three-dimensional structures.

However, in the case of technology of the prior art like that described above for extracting the amino acid sequence pattern of a protein, when attempting to extract a characteristic monosaccharide bonding pattern for the alignment of a local structure, since the interaction between hydroxyl group substituents and hydroxyl groups serving as acceptors of other monosaccharides bonded with α- or β-glucoside bonds by C1 hydroxyl groups involved in bonding between monosaccharides was not taken into consideration, enough data was unable to be obtained for elucidating a principle or developing a method for predicting polysaccharide three-dimensional structure. In addition, there was also the problem of the procedure being bothersome due to the susceptibility of the introduction of subjectivity involving detailed examination of three-dimensional diagrams and so forth when extracting characteristic monosaccharide bonding patterns.

Moreover, in the case of a method of the prior art, since the interaction of hydroxyl group substituents greatly contributes to the formation of polysaccharide three-dimensional structure in terms of monosaccharide bonding, although an examination was made as to whether or not interaction between substituents of hydroxyl groups that form local structures and their surrounding environment and hydroxyl groups serving as acceptors of other monosaccharides bonded with α- or β-glucoside bonds by C1 hydroxyl groups involved in bonding between monosaccharides is characteristic, polysaccharide characteristics were unable to be determined based on the output interaction patterns alone.

Therefore, in order to resolve these problems, the object of the present invention is to provide a method and device for extracting monosaccharide bonding patterns significant for elucidation of the formation of polysaccharide three-dimensional structure by judging whether or not a common pattern exists in the interaction between hydroxyl group substituents at each location of an alignment and hydroxyl groups serving as acceptors of other monosaccharides bonded with α- or β-glucoside bonds by C1 hydroxyl groups involved in bonding between monosaccharides when the alignment of a local structure is given.

In addition, another object of the present invention is to provide a method and device for extracting a monosaccharide bonding pattern significant for elucidating structure formation as well as extracting polysaccharides having a similar interaction pattern by judging whether or not a common pattern exists in the interaction between hydroxyl group substituents at each location of an alignment and hydroxyl groups serving as acceptors of other monosaccharides bonded with α- or β-glucoside bonds by C1 hydroxyl groups involved in bonding between monosaccharides when the alignment of a local structure, coordinate data relating to polysaccharides to which each local structure belongs, interaction patterns with the characteristic environment relating to polysaccharides to which each local structure belongs, and data on features and so forth are given.

Moreover, another object of the present invention is to provide a computer program and recording medium that are useful for carrying out this polysaccharide three-dimensional structure analysis method and device.

In addition, another object of the present invention is to provide a method for supporting diagnoses of diseases and so forth by evaluating polysaccharide-related functions when characteristics amounts of polysaccharide three-dimensional structure are given.

As a result of conducting extensive research in order to achieve the aforementioned objects, the inventor of this application found that, after having input the alignment of the local structures of a polysaccharide and coordinate data relating to polysaccharides to which each local structure belongs into a processing device, it is effective to detect interactions between hydroxyl group substituents in the local structures and hydroxyl groups serving as acceptors of other monosaccharides bonded with α- or β-glucoside bonds by C1 hydroxyl groups involved in bonding between monosaccharides based on the input data, evaluate the interaction patterns between hydroxyl group substituents and hydroxyl groups serving as acceptors of other monosaccharides bonded with α- or β-glucoside bonds by C1 hydroxyl groups involved in bonding between monosaccharides based on a formal classification of bonding relating to polysaccharides, extract those interaction patterns that appear in the local structures among each of the interaction patterns, and output those interaction patterns, thereby leading to completion of the present invention.

In one of its aspects, the present invention resides in a method for analyzing the three-dimensional structure of polysaccharides or sugar chains composed of monosaccharide linkages, comprising:
inputting the alignment of the local structures of a polysaccharide;
inputting coordinate data relating to polysaccharides to which each local structure belongs;
detecting the interactions between substituents of each hydroxyl group within a local structure and hydroxyl groups that serve as acceptors of other monosaccharides bonded by C1 hydroxyl groups involved in bonding between monosaccharides;
evaluating the interactions between hydroxyl group substituents and hydroxyl groups that serve as acceptors of other monosaccharides bonded with α- or β-glucoside bonds by C1 hydroxyl groups involved in bonding between monosaccharides, based on a formal classification of bonding relating to polysaccharides; and
outputting information relating to interactions with the environment that have a pattern which has been judged to be appearing in a local structure among each of the interaction patterns with the environment for each location of an alignment.

In addition, in another of its aspects, the present invention resides in a device for.analyzing the three-dimensional structure of polysaccharides composed of monosaccharide linkages, which comprises a data input device that inputs data, a data processing device that executes commands, a data management device that manages data, and a data output device that outputs data; wherein the data processing device comprises:
an alignment data input processing unit that inputs the alignments of polysaccharide local structures;
a coordinate data input processing unit that inputs coordinate data relating to polysaccharides to which each local structure belongs;
an interaction detection processing unit that detects an interaction between substituents of each hydroxyl group in a local structure and hydroxyl groups serving as acceptors of other monosaccharides bonded by C1 hydroxyl groups involved in bonding between monosaccharides;
an interaction pattern evaluation processing unit that evaluates patterns of interaction between hydroxyl group substituents and hydroxyl groups serving as acceptors of other monosaccharides bonded with α- or β-glucoside bonds by C1 hydroxyl groups involved in bonding between monosaccharides based on a formal classification of bonding relating to polysaccharides; and
an interaction pattern output processing unit that outputs information relating to interactions with the environment that have a pattern which has been judged to be appearing in a local structure among each of the interaction patterns with the environment for each location of an alignment.

Moreover, in still another of its aspects, the present invention resides in a polysaccharide three-dimensional structure analysis program used in combination with a computer when analyzing the three-dimensional structure of polysaccharides composed of monosaccharide linkages, for executing in the computer the following:
inputting the alignment of the local structures of a polysaccharide;
inputting coordinate data relating to polysaccharides to which each local structure belongs;
detecting the interactions between substituents of each hydroxyl group within a local structure and hydroxyl groups that serve as acceptors of other monosaccharides bonded by C1 hydroxyl groups involved in bonding between monosaccharides;
evaluating the interactions between hydroxyl group substituents and hydroxyl groups that serve as acceptors of other monosaccharides bonded with α- or β-glucoside bonds by C1 hydroxyl groups involved in bonding between monosaccharides, based on a formal classification of bonding relating to polysaccharides; and
outputting information relating to interactions with the environment that have a pattern which has been judged to be appearing in a local structure among each of the interaction patterns with the environment for each location of an alignment.

In addition to these inventions, the inventor of this application found that, if characteristic patterns of interaction with the environment are detected in common in local structures based on the alignment of local structures of a polysaccharide, and polysaccharides having similar interaction patterns are detected, then polysaccharides having similar interaction patterns can be extracted by detecting the bonding patterns of monosaccharides significant for elucidating the formation of polysaccharide three-dimensional structure and accumulating the polysaccharide interaction pattern data.

Thus, in still another of its aspects, the present invention resides in method for analyzing the three-dimensional structure of polysaccharides composed of monosaccharide linkages comprising:
inputting the alignment of the local structures of a polysaccharide;
inputting data on characteristic interaction patterns with the environment, features and so forth relating to polysaccharides to which each local structure belongs;
detecting the interactions between substituents of each hydroxyl group within a local structure and hydroxyl groups that serve as acceptors of other monosaccharides bonded by C1 hydroxyl groups involved in bonding between monosaccharides;
evaluating the interactions between hydroxyl group substituents and hydroxyl groups that serve as acceptors of other monosaccharides bonded with α- or β-glucoside bonds by C1 hydroxyl groups involved in bonding between monosaccharides, based on a formal classification of bonding relating to polysaccharides; and
outputting information relating to interactions with the environment that have a pattern which has been judged to be appearing in a local structure among each of the interaction patterns with the environment for each location of an alignment.

In addition, the present invention resides in a method for analyzing the three-dimensional structure of polysaccharides which, in combination with the aforementioned step in which data on characteristic interaction patterns with the environment, features and so forth relating to polysaccharides to which each local structure belongs is input in the aforementioned method, additionally comprises:
classifying and managing polysaccharide data;
analysising and processing data to detect polysaccharides having similar interaction patterns with respect to the resulting interaction patterns;
analysising, evaluating and processing data to evaluate polysaccharides having similar interaction patterns, based on a formal classification of bonding relating to polysaccharides; and
analysising data and output processing to output information in which polysaccharides have been judged to be similar in each of the interaction patterns.

Moreover, in still another of its aspects, the present invention resides in a device for analyzing the three-dimensional structure of polysaccharides composed of monosaccharide linkages which comprises a data input device that inputs data, a data processing device that executes commands, a data management device that manages data, and a data output device that outputs data; wherein the data processing device comprises:
an alignment data input processing unit that inputs the alignments of polysaccharide local structures;
a coordinate data input processing unit that inputs coordinate data relating to polysaccharides to which each local structure belongs;
another data input processing unit that inputs data on characteristic interaction patterns with the environment, features and so forth relating to polysaccharides to which each local structure belongs;
an interaction detection processing unit that detects an interaction between substituents of each hydroxyl group in a local structure and hydroxyl groups serving as acceptors of other monosaccharides bonded by C1 hydroxyl groups involved in bonding between monosaccharides;
an interaction pattern evaluation processing unit that evaluates patterns of interaction between hydroxyl group substituents and hydroxyl groups serving as acceptors of other monosaccharides bonded with α- or β-glucoside bonds by C1 hydroxyl groups involved in bonding between monosaccharides based on a formal classification of bonding relating to polysaccharides; and
an interaction pattern output processing unit that outputs information relating to interactions with the environment that have a pattern which has been judged to be appearing in a local structure among each of the interaction patterns with the environment for each location of an alignment.

In addition, the present invention resides in a device for analyzing the three-dimensional structure of polysaccharides which, in combination with the aforementioned other data input processing unit that inputs data on characteristic interaction patterns with the environment, features and so forth relating to polysaccharides to which each local structure belongs in the aforementioned device, additionally comprises:
a polysaccharide classification and management unit that classifies and manages polysaccharide data;
a data analysis processing unit that detects polysaccharides having similar interaction patterns with respect to the resulting interaction patterns;
a data analysis evaluation processing unit that evaluates polysaccharides having similar interaction patterns based on a formal classification of bonding relating to polysaccharides; and
a data analysis output processing unit that outputs information in which polysaccharides have been judged to be similar in each of the interaction patterns.

Moreover, in still another of its aspects, the present invention resides in a polysaccharide three-dimensional structure analysis program used in combination with a computer when analyzing the three-dimensional structure of polysaccharides composed of the following monosaccharide linkages, for executing in the computer the following:
inputting the alignment of the local structures of a polysaccharide;
inputting data on characteristic interaction patterns with the environment, features and so forth relating to polysaccharides to which each local structure belongs;
detecting the interactions between substituents of each hydroxyl group within a local structure and hydroxyl groups that serve as acceptors of other monosaccharides bonded by C1 hydroxyl groups involved in bonding between monosaccharides;
evaluating the interaction patterns between hydroxyl group substituents and hydroxyl groups that serve as acceptors of other monosaccharides bonded with α- or β-glucoside bonds by C1 hydroxyl groups involved in bonding between monosaccharides, based on a formal classification of bonding relating to polysaccharides; and
outputting information relating to interactions with the environment that have a pattern which has been judged to be appearing in a local structure among each of the interaction patterns with the environment for each location of an alignment.

A polysaccharide three-dimensional structure analysis program according to the present invention is preferably a polysaccharide three-dimensional structure analysis program which, in combination with the aforementioned step in which data on characteristic interaction patterns with the environment, features and so forth relating to polysaccharides to which each local structure belongs is input, additionally comprises:
classifying and managing polysaccharide data;
analysising and processing data to detect polysaccharides having similar interaction patterns with respect to the resulting interaction patterns;
analysising, evaluating and processing data to evaluate polysaccharides having similar interaction patterns, based on a formal classification of bonding relating to polysaccharides; and
analysising data and output processing to output information in which polysaccharides have been judged to be similar in each of the interaction patterns.

Moreover, the present invention resides in a recording medium used in combination with a computer when analyzing the three-dimensional structure of polysaccharides composed of monosaccharide linkages which contains the aforementioned polysaccharide three-dimensional structure analysis program explained later in detail, and which can be read by a computer.

In addition, as a result of conducting extensive research to achieve the aforementioned object of providing diagnostic support, the inventor of this application found that after inputting polysaccharide information, patient symptoms, inputting existing patient information and so forth into a processing device, extracting features possessed by polysaccharide three-dimensional structure based on that input data, evaluating polysaccharide function, evaluating pathology, treatment and prevention based on information relating to polysaccharides and then outputting that information is effective for providing diagnostic support by using the three-dimensional structures of polysaccharides, thereby leading to completion of the present invention.

The present invention, in still another of its aspects, resides in a method for supporting diagnoses of diseases and so forth using the three-dimensional structures of polysaccharides composed of monosaccharide linkages, comprising:
inputting polysaccharide information;
inputting patient symptoms;
inputting patient existing information;
extracting the features possessed by polysaccharide three-dimensional structures;
evaluating pathology based on polysaccharide information;
evaluating suitable treatment;
evaluating suitable prevention;
outputting information relating to determination of pathology;
outputting information relating to suitable treatment; and
outputting information relating to suitable prevention.

In addition, in still another of its aspects, the present invention resides in a method for supporting diagnoses of diseases and so forth using the three-dimensional structures of polysaccharides composed of monosaccharide linkages, comprising:
evaluating pathology based on polysaccharide information;
evaluating suitable treatment;
evaluating suitable prevention;
outputting information relating to determination of pathology is output;
outputting information relating to suitable treatment; and
outputting information relating to suitable prevention.

### BRIEF DESCRIPTION OF THE DRAWINGS

Fig. 1 is a flow chart explaining the principle of a polysaccharide three-dimensional structure analysis method in one aspect of the present invention;
Fig. 2 is an explanatory drawing showing an example of the composition of a polysaccharide applicable for analysis in the present invention;
Fig. 3 is an explanatory drawing showing an embodiment of a polysaccharide three-dimensional structure analysis method according to the present invention;
Fig. 4 is an explanatory drawing showing an example of alignment of a local structure used in carrying out the present invention;
Fig. 5 is an explanatory drawing showing an example of coordinate data of a local structure used in carrying out the present invention;
Fig. 6 is a schematic drawing showing an interaction pattern that can be confirmed in carrying out the present invention;
Fig. 7 is an explanatory drawing showing an example of an output obtained in carrying out the present invention;
Fig. 8 is a flow chart explaining the principle of a polysaccharide three-dimensional structure analysis method in another aspect of the present invention;
Fig. 9 is a processing explanatory drawing of an example of the present invention;
Fig. 10 is a drawing showing an example of an alignment of a local structure according an example of the present invention;
Fig. 11 is a graph showing an example of physical quantities in an embodiment of the present invention;
Fig. 12 is a schematic drawing showing an example of an output according to an embodiment of the present invention;
Fig. 13 is a schematic drawing showing an example of an output according to an embodiment of the present invention;
Fig. 14 is a perspective view schematically showing a method and device for installing a program used in a polysaccharide three-dimensional structure analysis method and device of the present invention, along with its recording medium, in a monitor;
Fig. 15 is a block diagram of the inside of the monitor shown in Fig. 14;
Fig. 16 is a flow chart explaining the principle of a diagnostic support method according to the present invention;
Fig. 17 is a flow chart showing a preferable embodiment of a diagnostic support method according to the present invention;
Fig. 18 is a flow chart of the processing in the embodiment shown in Fig. 17;
Fig. 19 is an explanatory drawing showing an example of a polysaccharide composition that can be input by a polysaccharide data input processing section in carrying out the present invention;
Fig. 20 is a graph showing an example of an output (feature) according to an embodiment of the present invention; and
Fig. 21 is an explanatory drawing of a neural network for identifying IgA nephropathy patients used in a diagnostic support method of the prior art.

### BEST MODE FOR CARRYING OUT THE INVENTION

The present invention can be advantageously carried out in various embodiments explained with reference to the following drawings. Furthermore, the present invention is not to be considered to be limited to the following embodiments, and various modifications and improvements can be made within the scope of the present invention.

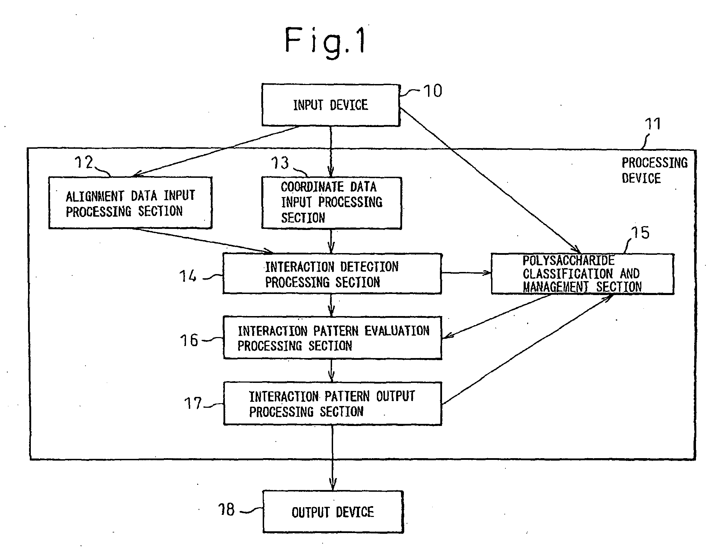

Fig. 1 is a flow chart explaining the principle of a polysaccharide or sugar chain three-dimensional structure analysis method and device in a first aspect of the present invention. Data input device 10 is a device that inputs data to a data processing device 11, and includes, for example, an external storage device and a keyboard device. Data processing device 11 is provided with a CPU, memory and so forth, and executes a program that analyzes the three-dimensional structure of polysaccharides composed of monosaccharide linkages.

In data processing apparatus 11, an alignment data input processing section 12 is a processing means that inputs information which defines alignments of the local structures of a polysaccharide, namely the substituents of each hydroxyl group of each local structure and hydroxyl groups that serve as acceptors of other monosaccharides bonded by C1 hydroxyl groups involved in monosaccharide bonding.

In addition, a coordinate data input processing section 13 is a processing means that inputs polysaccharide coordinate data. Coordinate data defines, for example, coordinates in three-dimensional space as well as the numbers of the substituents of hydroxyl groups belonging to atoms that compose the polysaccharide along with hydroxyl groups serving as acceptors of other monosaccharides bonded with α- or β-glucoside bonds by C1 hydroxyl groups involved in bonding between monosaccharides, for each atom that composes the polysaccharide.

Interaction detection processing section 14 is a processing means that detects interactions between substituents of each hydroxyl group in a local structure and hydroxyl groups serving as acceptors of other monosaccharides bonded with α- or β-glucoside bonds by C1 hydroxyl groups involved in bonding between monosaccharides. Here, interactions are detected in the form of interactions between substituents of hydroxyl groups within a local structure and hydroxyl groups serving as acceptors of other monosaccharides bonded with α- or β-glucoside bonds by C1 hydroxyl groups involved in bonding between monosaccharides.

Polysaccharide classification and management section 15 is a means that makes a formal classification of bonding relating to polysaccharides as well as retains polysaccharide classification information determined later, and manages information that defines the grouping of monosaccharides that exhibit a certain specific property. Namely, polysaccharide classification and management processing section 15 can be referred to as a database.

Interaction pattern evaluation processing section 16 is a processing means that evaluates the interaction patterns between hydroxyl group substituents and hydroxyl groups serving as acceptors of other monosaccharides bonded with α- or β-glucoside bonds by C1 hydroxyl groups involved in bonding between monosaccharides based on classifications relating to polysaccharides in polysaccharide classification and management processing section 15, or evaluates and extracts those interaction patterns with the environment that appear in local structures for each location of an alignment.

Interaction pattern output processing section 17 is a processing means that outputs information relating to interactions with the environment that have a pattern extracted by interaction pattern evaluation processing section 16 to a data output device 18.

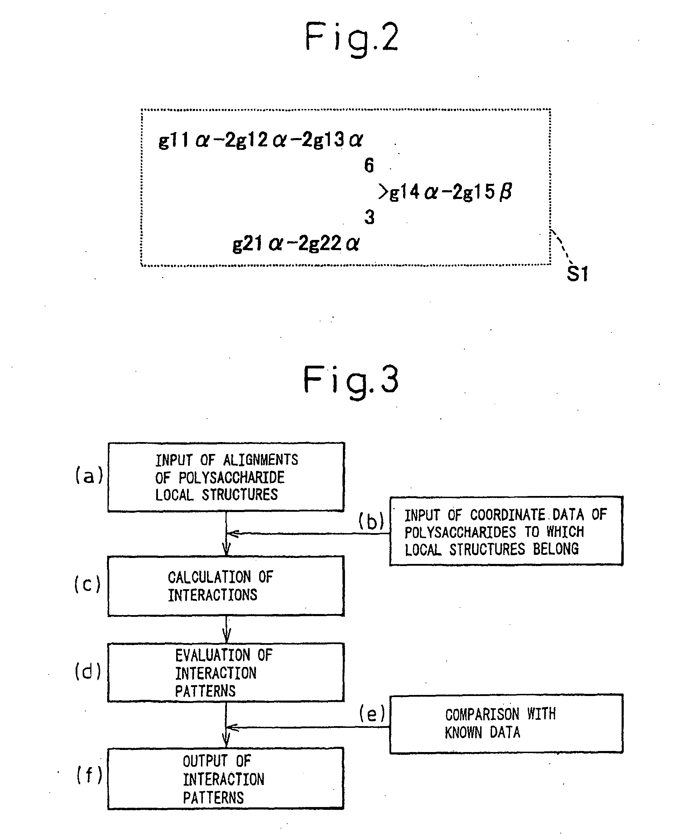

The flow of polysaccharide or sugar chain three-dimensional structure analysis according to the present invention as described above can be easily understood from Fig. 2 which shows the local structure of a double-chain compound or complex polysaccharide. In Fig. 2, gi represents a monosaccharide, ri a hydroxyl group substituent, and Si a polysaccharide local structure (i = 1).

As shown in Fig. 2, a polysaccharide is a linear molecule formed by the bonding of a large number of monosaccharides. Since monosaccharide hydroxyl groups are substituted or a large number hydroxyl groups that serve as acceptors of other monosaccharides bonded with α- or β-glucoside bonds by C1 hydroxyl groups involved in bonding between monosaccharides are present, their structure is diverse. In actuality, in addition to the illustrated double-chain complex polysaccharide, polysaccharides also include high-mannose types, mannose-6-phosphate types, complex types, single-chain complex types, triple-chain complex types, quadruple-chain complex types, diallyl Lex types and bisecting complex types.

In these polysaccharides, since polysaccharide function is expressed due to the formation of specific three-dimensional structures by the polysaccharides, it would be desirable to elucidate the mechanism by which these three-dimensional structures are formed so as to, for example, be able to predict the three-dimensional structure of a polysaccharide from the bonding of a certain monosaccharide.

One possible method that is considered to be effective for elucidating the mechanism by which three-dimensional structures are formed is to extract and analyze specific monosaccharide linkages from local structural patterns that appear in common in several polysaccharides. An important characteristic of the present invention is that it not only considers monosaccharide linkages within a local structure when extracting a characteristic monosaccharide bonding pattern, but also considers the interaction between substituents of hydroxyl groups present in the vicinity and hydroxyl groups serving as acceptors of other monosaccharides bonded with α- or β-glucoside bonds by C1 hydroxyl groups involved in bonding between monosaccharides.

For example, the local structures S1, S2 and S3 having a similar structure are assumed to be present in three polysaccharides. In such a case, the alignments of these three types of local structures are input by alignment data input processing section 12. In addition, coordinate data of each polysaccharide is input by coordinate data input processing section 13. The interaction with hydroxyl groups serving as acceptors of other monosaccharides bonded with α- or β-glucoside bonds by C1 hydroxyl groups involved in bonding between monosaccharides is detected for each hydroxyl group substituent of local structures S1, S2 and S3. This detection processing is carried out with interaction detection processing section 14.

According to the present invention, interactions between each hydroxyl group substituent of polysaccharide local structures and hydroxyl groups serving as acceptors of other monosaccharides bonded with α- or β-glucoside bonds by C1 hydroxyl groups involved in bonding between monosaccharides, and their patterns, can be determined by a clearly defined specific procedure, and after the alignments of the local structures in question and coordinate data of the polysaccharide to which these local structures belong are input, the results can be output automatically without user intervention.

Next, a preferred embodiment of a polysaccharide three-dimensional structure analysis method according to the present invention is explained in order while referring to Fig. 3.

### Step (a):

N number of polysaccharide local structure alignments are input by alignment input processing section 12. Here, N is an arbitrary integer of 2 or more. An alignment refers to that which defines substituents of each hydroxyl group of each local structure and hydroxyl groups serving as acceptors of other monosaccharides bonded with α- or β-glucoside bonds by C1 hydroxyl groups involved in bonding between monosaccharides.

### Step (b):

The coordinate data of a polysaccharide to which N number of local structures belong is input by coordinate data input processing section 13. Coordinate data refers to that which defines coordinates in three-dimensional space for each atom that composes the polysaccharide, as well as the numbers of substituents of each hydroxyl group belonging to those atoms and hydroxyl groups serving as acceptors of other monosaccharides bonded with α- or β-glucoside bonds by C1 hydroxyl groups involved in bonding between monosaccharides. In addition, if necessary, in the evaluation step described to follow, definition information relating to the types of atoms or the types of substituents of the hydroxyl groups to which they belong and other data may also be input. Moreover, this coordinate data input step may be omitted it if can be substituted by calculations.

### Step (c):

Hydroxyl groups serving as acceptors of other monosaccharides bonded with α- or β-glucoside bonds by C1 hydroxyl groups involved in bonding between monosaccharides are detected for substituents of each hydroxyl group of each local structure by interaction detection processing section 14. Furthermore, in the present invention, pairs of hydroxyl group substituents and hydroxyl groups serving as acceptors of other monosaccharides bonded with α- or β-glucoside bonds by C1 hydroxyl groups involved in bonding between monosaccharides are referred to as "interactions between those hydroxyl group substituents and hydroxyl groups serving as acceptors of other monosaccharides bonded with α- or β-glucoside bonds by C1 hydroxyl groups involved in bonding between monosaccharides".

Here, interactions between substituents rij of each hydroxyl group in each local structure Si and hydroxyl groups serving as acceptors of other monosaccharides bonded with α- or β-glucoside bonds by C1 hydroxyl groups involved in bonding between monosaccharides are determined with respect to hydroxyl group substituents rij and hydroxyl groups serving as acceptors of other monosaccharides bonded with α- or β-glucoside bonds by C1 hydroxyl groups involved in bonding between monosaccharides.

### Step (d):

Interaction pattern evaluation processing section 16 defines patterns of interaction between substituents of each hydroxyl group in local structures and hydroxyl groups serving as acceptors of other monosaccharides bonded with α- or β-glucoside bonds by C1 hydroxyl groups involved in bonding between monosaccharides detected in step (c) using polysaccharide classifications and known interaction patterns managed with polysaccharide classification and management section 15. In the case a novel pattern has been recognized, it is added as new data to polysaccharide classification and management section 15. Polysaccharide classification refers to that which defines groups of monosaccharides A1, A2 and so forth that exhibit a certain specific property. Which properties are considered and the number of groups defined are arbitrary.

For example, an interaction between a substituent rij of a hydroxyl group of a certain local structure Si and a hydroxyl group serving as an acceptor of another monosaccharide bonded with an α- or β-glucoside bond by a C1 hydroxyl group involved in bonding between monosaccharides is assumed to be detected by step (c). Here, a is a hydroxyl group serving as an acceptor of another monosaccharide bonded with an α- or β-glucoside bond by a C1 hydroxyl group involved in bonding between monosaccharides. In the classification of polysaccharides, rij is an acetyl group (Ac) such as N-acetylglucosan (GlcNAc), a carboxyl group-substituted form (cA) such as glucuronic acid (GlcA) or a neuraminic acid derivative (Neu) such as N-acetylneuraminic acid (NeuAc), while a is one of four types consisting of C2, C3, C4 and C6.

Moreover, interaction patterns observed in Nt or more among N number of local structures are detected for each location of the alignments input in step (a). Here, Nt is an integer of 1 to N, and is a parameter that is arbitrarily defined by the user.

### Steps (e) and (f):

Interaction pattern output processing section 17 sends interactions having a pattern detected in step (c) to polysaccharide classification and management section 15 or outputs them to a printer, display or external storage device and so forth for each location of the alignments.

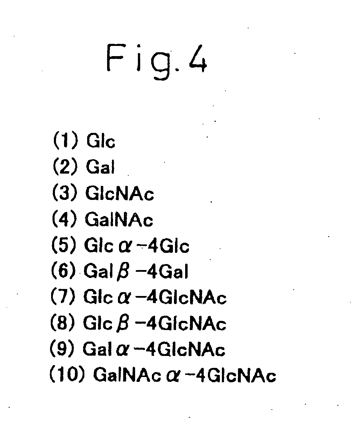

Fig. 4 shows an example of an alignment of a local structure that can be input by the alignment input processing section in carrying out the present invention. Here, Glc represents glucose, Gal galactose, NAc an N-acetyl group, α an α-glucoside group, and β a β-glucoside group, while "-4" in the formula means that the hydroxyl group serving as an acceptor of another bonded monosaccharide is C4.

There are no particular limitations on the notation method of alignments input to the alignment input processing section. For example, any notation method may be used provided it defines the substituents of hydroxyl groups in local structures and hydroxyl groups serving as acceptors of other monosaccharides bonded with α- or β-glucoside bonds by C1 hydroxyl groups involved in bonding between monosaccharides. Furthermore, in Fig. 4, the monosaccharide species of the substituents of each hydroxyl group are represented by arranging in a matrix format in accordance with methods ordinarily used in the field of structural analysis. Each row of the matrix indicates monosaccharide bonding of each local structure, while each column indicates the corresponding relationship of hydroxyl group substituents between the local structures.

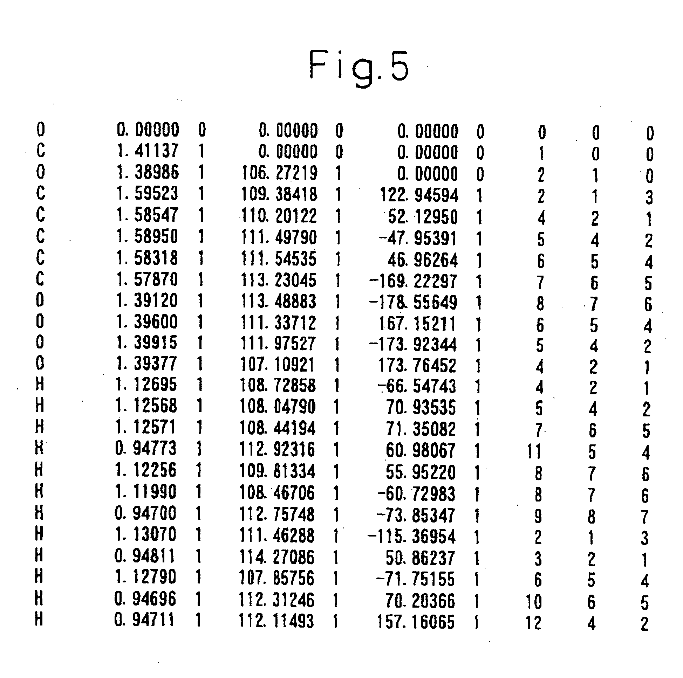

Fig. 5 shows an example of coordinate data of a local structure that can be input by the coordinate data input processing section in carrying out the invention. Furthermore, the coordinate data referred to here uses structural data from various databases. For example, since crystal coordinate data of X-ray crystal diffraction of the Protein Data Bank (PDB) has high atomic resolution and does not contain information on hydrogen atoms (H), it can be used by adding hydrogen atoms using molecular modeling software, and coordinate data has been produced and used in the present aspect as well by using this data software.

Evaluation of interactions between hydroxyl group substituents and hydroxyl groups serving as acceptors of other monosaccharides bonded with α- or β-glucoside bonds by C1 hydroxyl groups involved in bonding between monosaccharides detected by interaction detection processing section 14 can be carried out according to various procedures. For example, evaluation work can be carried out advantageously according to the procedure indicated below.

The dipolar moment calculated from the molecular orbit method is used an example of representing interactions. In addition, free energy calculated from the molecular dynamics method can be used, dipolar moment for hydrophobicity and electrostatic potential for hydrophilicity can be used calculated from the molecular orbit method, or reactivity can be investigated based on the properties of frontier orbitals in order to predict reactions in systems having small molecular polarization.

Here, the units of the aforementioned dipolar moment are debyes. Furthermore, in the present embodiment, from the standpoint of focusing on interactions between hydroxyl group substituents and hydroxyl groups serving as acceptors of other monosaccharides bonded with α- or β-glucoside bonds by C1 hydroxyl groups involved in bonding between monosaccharides, only the calculation of hydroxyl group substituents and hydroxyl groups serving as acceptors of other monosaccharides bonded with α- or β-glucoside bonds by C1 hydroxyl groups involved in bonding between monosaccharides.is taken into consideration.

An example of determining the interaction between substituents of hydroxyl groups of a local structure in question and hydroxyl groups serving as acceptors of other monosaccharides bonded with α- or β-glucoside bonds by C1 hydroxyl groups involved in bonding between monosaccharides using the aforementioned procedure is indicated below.

Galactose demonstrates a higher dipolar moment than glucose and has high reactivity. If an N-acetyl group is introduced as a hydroxyl group substituent, it demonstrates a high dipolar moment and has high reactivity.

The number of substituents of hydroxyl groups in a local structure, the types of monosaccharides, the mode of bonding with α- or β-glucoside bonds by hydroxyl groups by C1 hydroxyl groups involved in bonding between monosaccharides, and the structural activity correlation with hydroxyl groups serving as acceptors of other monosaccharides bonded serves as the basic data.

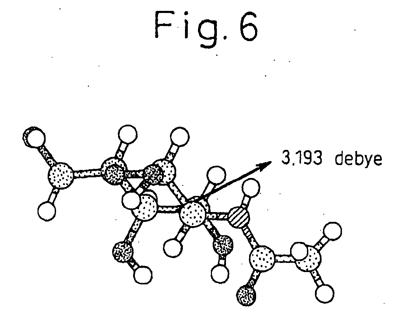

Fig. 6 is a schematic drawing showing which type of interaction pattern is possible.

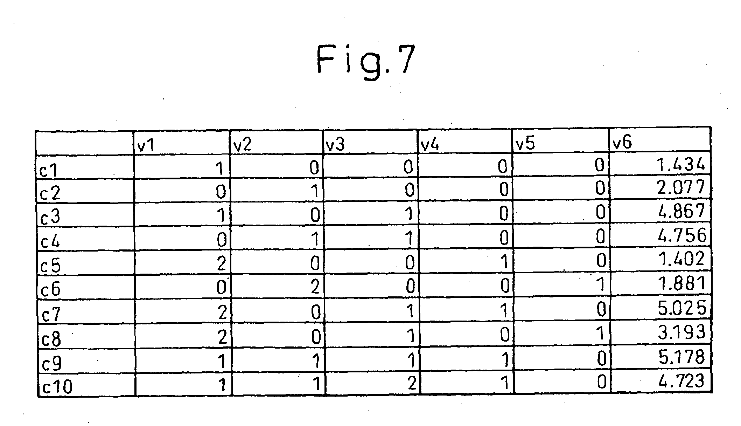

In addition, Fig. 7 shows an example of an output according to one example of the present invention. In this example, in the case there were interaction patterns with the environment appearing in all ten local structures with respect to each location of alignments (1) through (10) shown in Fig. 4, an interaction with the environment having that pattern is output. For example, the pattern appearing in the seventh piece of data indicates that glucose is bonded to the 4 location of glucose in which an N-acetyl group has been introduced with an α-glucoside bond.

Polysaccharide coordinate data is not limited to only that previously described. For example, arbitrary data assigned by a user may be input as coordinate data. In addition, which properties of polysaccharides are considered and how many groups are defined in classifying monosaccharides is arbitrary. This monosaccharide classification information may be made to be input dynamically from an input device 10 shown in Fig. 1.

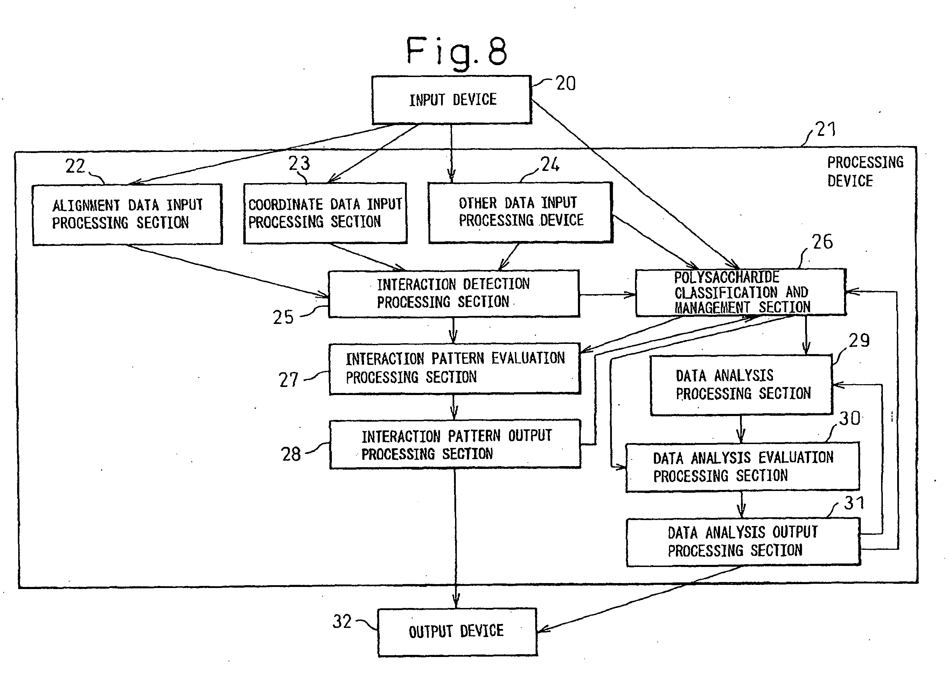

Fig. 8 is a flow chart explaining the principle of a polysaccharide three-dimensional structure analysis method and device in a second embodiment of the present invention. Input device 20 is a device that inputs data such as an external storage device or keyboard device. Processing device 21 is a device equipped with a CPU and memory that executes a program that analyses the three-dimensional structure of polysaccharides composed of monosaccharide linkages. Alignment input processing section 22 is a processing means that inputs information which defines alignments of the local structures of a polysaccharide, namely the substituents of each hydroxyl group of each local structure and hydroxyl groups that serve as acceptors of other monosaccharides bonded by C1 hydroxyl groups involved in monosaccharide bonding. A coordinate data input processing section 23 is a processing means that inputs polysaccharide coordinate data. Coordinate data defines, for example, coordinates in three-dimensional space as well as the numbers of the substituents of hydroxyl groups belonging to atoms that compose the polysaccharide along with hydroxyl groups serving as acceptors of other monosaccharides bonded with α- or β-glucoside bonds by C1 hydroxyl groups involved in bonding between monosaccharides, for each atom that composes the polysaccharide. Other dada input processing section 24 is a processing means that inputs polysaccharide data (excluding data 22 and 23). The "other data" referred to here is, for example, information on known polysaccharide interaction patterns.

In addition, an interaction detection processing section 25 is a processing means that detects interactions between substituents of each hydroxyl group in a local structure and hydroxyl groups serving as acceptors of other monosaccharides bonded with α- or β-glucoside bonds by C1 hydroxyl groups involved in bonding between monosaccharides. Here, interactions are detected in the form of interactions between substituents of hydroxyl groups within a local structure and hydroxyl groups serving as acceptors of other monosaccharides bonded with α- or β-glucoside bonds by C1 hydroxyl groups involved in bonding between monosaccharides.

A polysaccharide classification and management section 26 is a means that makes a formal classification of bonding relating to polysaccharides as well as retains polysaccharide classification information determined later, and manages information that defines the grouping of monosaccharides that exhibit a certain specific property.

In addition, an interaction pattern evaluation processing section 27 is a processing means that evaluates the interaction patterns between hydroxyl group substituents and hydroxyl groups serving as acceptors of other monosaccharides bonded with α- or β-glucoside bonds by C1 hydroxyl groups involved in bonding between monosaccharides based on the classifications relating to polysaccharides, or evaluates and extracts those interaction patterns with the environment that appear in local structures for each location of an alignment.

In addition, an interaction pattern output processing section 28 is a processing means that outputs information relating to interactions with the environment that have a common pattern extracted by interaction pattern evaluation processing section 27 to an output device 32.

Moreover, a data analysis processing section 29 is a means for detecting polysaccharides having a similar interaction pattern with respect to interaction patterns obtained with interaction pattern output processing section 29.

A data analysis evaluation processing section 30 is a processing means that evaluates and extracts polysaccharides having a similar interaction pattern based on a formal classification of bonding relating to polysaccharides.

A data analysis output processing section 31 is a processing means that outputs to output device 32 polysaccharide group information of candidates having similar functions that have been extracted by data analysis evaluation processing section 30 and judged to be similar polysaccharides. In addition, data analysis output processing section 31 is able to accumulate that information in polysaccharide classification and management section 26. In the case similar polysaccharides have not been determined with data analysis output processing section 31, analysis can be repeated by changing the data analysis processing conditions with data analysis processing section 29.

The operation of the present invention can be easily understood from the previously explanation with reference to Fig. 2. Namely, as shown in Fig. 2, a polysaccharide is a linear molecule formed by the bonding of a large number of monosaccharides. Since monosaccharide hydroxyl groups are substituted or a large number hydroxyl groups that serve as acceptors of other monosaccharides bonded with α- or β-glucoside bonds by C1 hydroxyl groups involved in bonding between monosaccharides are present, their structure is diverse. Since polysaccharide function is expressed due to the formation of specific three-dimensional structures by the polysaccharides, it would be desirable to elucidate the mechanism by which these three-dimensional structures are formed so as to, for example, be able to predict the three-dimensional structure of a polysaccharide from the bonding of a certain monosaccharide.

One possible method that is considered to be effective for elucidating the mechanism by which three-dimensional structures are formed is to extract and analyze specific monosaccharide bonding patterns from local structural patterns that appear in common in several polysaccharides. The present invention is characterized in that it not only considers monosaccharide bonding within a local structure when extracting a characteristic monosaccharide bonding pattern, but also considers the interaction between substituents of hydroxyl groups present in the vicinity and hydroxyl groups serving as acceptors of other monosaccharides bonded with α- or β-glucoside bonds by C1 hydroxyl groups involved in bonding between monosaccharides.

For example, the local structures S1, S2 and S3 having a similar structure are assumed to be present in three polysaccharides. In such a case, the alignments of these three types of local structures are input by alignment data input processing section 22. In addition, coordinate data of each polysaccharide is input by coordinate data input processing section 23. The interaction with hydroxyl groups serving as acceptors of other monosaccharides bonded with α- or β-glucoside bonds by C1 hydroxyl groups involved in bonding between monosaccharides is detected for each hydroxyl group substituent of local structures S1, S2 and S3.

According to the present invention, interactions between each hydroxyl group substituent of polysaccharide local structures and hydroxyl groups serving as acceptors of other monosaccharides bonded with α- or β-glucoside bonds by C1 hydroxyl groups involved in bonding between monosaccharides, and their patterns, can be determined by a clearly defined specific procedure, and after the alignments of the local structures in question and coordinate data of the polysaccharide to which these local structures belong are input, the results can be output automatically without user intervention.

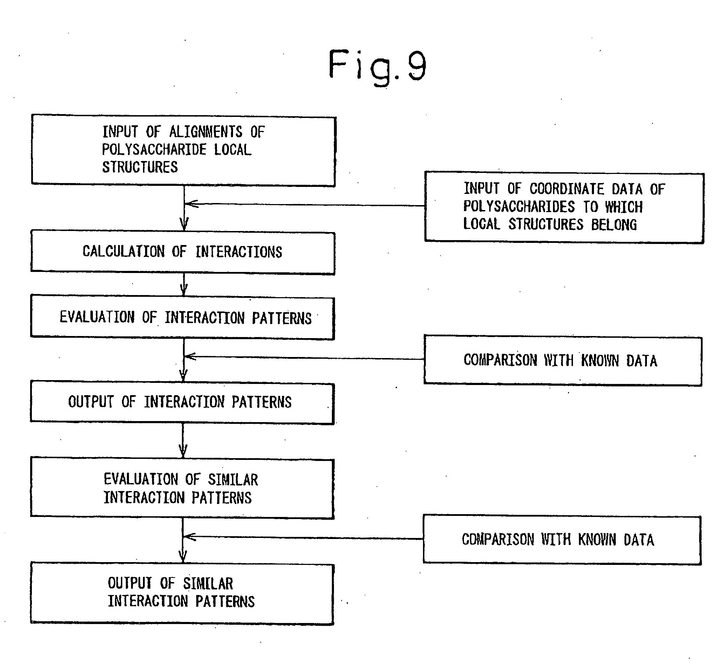

Fig. 9 is a processing explanatory drawing of an example of the present invention. The following provides an explanation of a series of processing steps (1) through (10) with reference to this drawing.

### Step (1):

N number of polysaccharide local structure alignments are input by alignment input processing section 22. Here, N is an arbitrary integer of 2 or more. An alignment refers to that which defines substituents of each hydroxyl group of each local structure and hydroxyl groups serving as acceptors of other monosaccharides bonded with α- or β-glucoside bonds by C1 hydroxyl groups involved in bonding between monosaccharides.

### Step (2):

The coordinate data of a polysaccharide to which N number of local structures belong is input by coordinate data input processing section 23. Coordinate data refers to that which defines coordinates in three-dimensional space for each atom that composes the polysaccharide, as well as the numbers of substituents of each hydroxyl group belonging to those atoms and hydroxyl groups serving as acceptors of other monosaccharides bonded with α- or β-glucoside bonds by C1 hydroxyl groups involved in bonding between monosaccharides. Moreover, polysaccharide data (excluding the aforementioned data 22 and 23) is input by other data input processing section 24. Other data is, for example, information on known polysaccharide interaction patterns, definition information relating to the types of atoms or the types of substituents of the hydroxyl groups to which they belong.

### Step (3):

Hydroxyl groups serving as acceptors of other monosaccharides bonded with α- or β-glucoside bonds by C1 hydroxyl groups involved in bonding between monosaccharides are detected for substituents of each hydroxyl group of each local structure by interaction detection processing section 25. Here, pairs of hydroxyl group substituents and hydroxyl groups serving as acceptors of other monosaccharides bonded with α- or β-glucoside bonds by C1 hydroxyl groups involved in bonding between monosaccharides are referred to as "interactions between those hydroxyl group substituents and hydroxyl groups serving as acceptors of other monosaccharides bonded with α- or β-glucoside bonds by C1 hydroxyl groups involved in bonding between monosaccharides". Here, interactions between substituents rij of each hydroxyl group in each local structure Si and hydroxyl groups serving as acceptors of other monosaccharides bonded with α- or β-glucoside bonds by C1 hydroxyl groups involved in bonding between monosaccharides are determined with respect to hydroxyl group substituents rij and hydroxyl groups serving as acceptors of other monosaccharides bonded with α- or β-glucoside bonds by C1 hydroxyl groups involved in bonding between monosaccharides.

### Step (4):

Interaction pattern evaluation processing section 27 defines patterns of interaction between substituents of each hydroxyl group in local structures and hydroxyl groups serving as acceptors of other monosaccharides bonded with α- or β-glucoside bonds by C1 hydroxyl groups involved in bonding between monosaccharides detected in the aforementioned processing step (3) using polysaccharide classifications and known interaction patterns managed with polysaccharide classification and management section 26. New patterns are added to the polysaccharide classification and management section. Polysaccharide classification refers to that which defines groups of monosaccharides A1, A2 and so forth that exhibit a certain specific property. Which properties are considered and the number of groups defined are arbitrary.

For example, an interaction (rij, a) between a substituent rij of a hydroxyl group of a certain local structure Si and a hydroxyl group serving as an acceptor of another monosaccharide bonded with an α- or β-glucoside bond by a C1 hydroxyl group involved in bonding between monosaccharides is assumed to be detected by processing step (3). Here, a is a hydroxyl group serving as an acceptor of another monosaccharide bonded with an α- or β-glucoside bond by a C1 hydroxyl group involved in bonding between monosaccharides. In the classification of polysaccharides, rij is an acetyl group (Ac) such as N-acetylglucosan (GlcNAc), a carboxyl group-substituted form (cA) such as glucuronic acid (GlcA) or a neuraminic acid derivative (Neu) such as N-acetylneuraminic acid (NeuAc), while a is one of four types consisting of C2, C3, C4 and C6.

### Step (5):

Moreover, interaction patterns observed in Nt or more among N number of local structures are detected for each location of the alignments input in processing step (1). Nt is an integer of 1 to N, and is a parameter that is arbitrarily defined by the user.

### Step (6):

Interaction pattern output processing section 28 sends interactions having a pattern detected in step (5) to polysaccharide classification and management section 26 or outputs them to a printer, display or external storage device and so forth for each location of the alignments.

### Step (7):

Polysaccharides having similar interaction patterns are detected by data analysis processing section 29.

### Step (8):

Data analysis evaluation processing section 30 extracts those polysaccharides having similar interaction patterns between substituents of each hydroxyl group in the local structures and hydroxyl groups serving as acceptors of other monosaccharides bonded with α- or β-glucoside bonds by C1 hydroxyl groups involved in bonding between monosaccharides detected in processing step (7) using polysaccharide classifications and existing interaction patterns managed with polysaccharide classification and management section 26. Similar patterns are added to the polysaccharide classification and management section. Classification of similar polysaccharides defines groups of monosaccharides B1, B2 and so forth that exhibit a certain specific similar property. Which properties are considered and the number of groups defined are arbitrary.

### Step (9):

For example, a local structure Si1 having a physical quantity that is similar to a certain local structure Si is assumed to be detected in processing step (7). Polysaccharides having similar physical quantity patterns detected in processing step (8) are sent to polysaccharide classification and management section 26 or output to an output device 32 such as a printer, display or external storage device for each location of an alignment by data analysis output processing section 31.

### Step (10):

Data analysis output processing section 31 re-detects polysaccharides having similar interaction patterns by returning a certain local structure Si to data analysis processing section 29 in the case a polysaccharide having a similar physical quantity pattern is not detected.

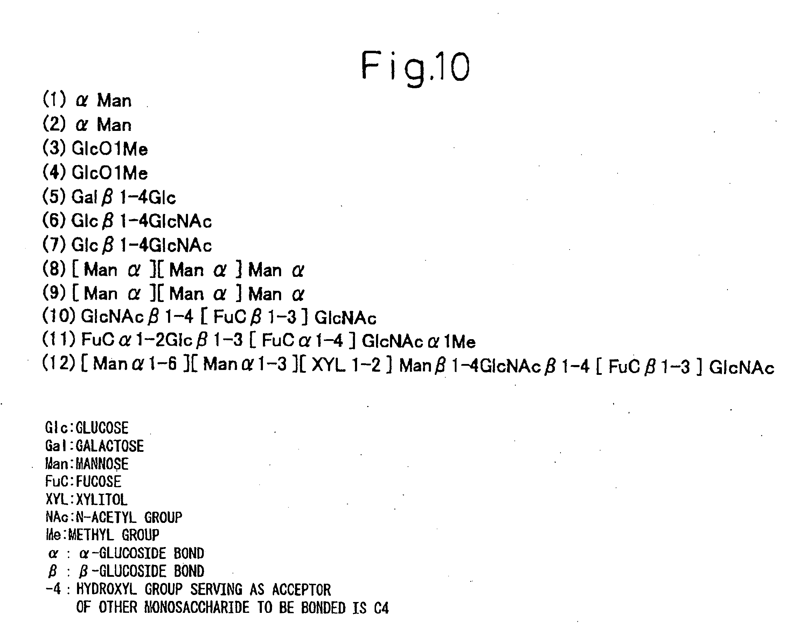

Fig. 10 is a drawing showing an example of an alignment of a local structure that is input by the alignment input processing section according to an example of the present invention. Here, the local structures in question consist of 12 local structures.

Although any notation method may be used for the notation method of the input alignments provided it defines the substituents of hydroxyl groups in local structures and hydroxyl groups serving as acceptors of other monosaccharides bonded with α- or β-glucoside bonds by C1 hydroxyl groups involved in bonding between monosaccharides. Here, monosaccharide species of the substituents of each hydroxyl group are represented by arranging in a matrix format in accordance with normally used methods. Each row of the matrix indicates monosaccharide bonding of each local structure, while each column indicates the corresponding relationship of hydroxyl group substituents between the local structures.

The previous example of coordinate data explained with reference to Fig. 5 can also used as an example of local structure coordinate data that is input by the coordinate data input processing section according to the aforementioned example of the present invention. As was previously described, since crystal coordinate data of X-ray crystal diffraction of the Protein Data Bank has high atomic resolution and does not contain information on hydrogen atoms (H), it can be used as coordinate data by adding hydrogen atoms using molecular modeling software.

Evaluation of interactions between hydroxyl group substituents and hydroxyl groups serving as acceptors of other monosaccharides bonded with α- or β-glucoside bonds by C1 hydroxyl groups involved in bonding between monosaccharides detected by interaction detection processing section 25 was carried out according to various procedures in the present example.

The dipolar moment calculated from the molecular orbit method and the free energy calculated from the molecular dynamics method were used as examples of representing interactions. In addition, dipolar moment for hydrophobicity and electrostatic potential for hydrophilicity can be used calculated from the molecular orbit method, or reactivity can be investigated based on the properties of frontier orbitals in order to predict reactions in systems having small molecular polarization.

Here, the units are debyes or kcal/mole. Furthermore, in the present example; from the standpoint of focusing on interactions between hydroxyl group substituents and hydroxyl groups serving as acceptors of other monosaccharides bonded with α- or β-glucoside bonds by C1 hydroxyl groups involved in bonding between monosaccharides, only the calculation of hydroxyl group substituents and hydroxyl groups serving as acceptors of other monosaccharides bonded with α- or β-glucoside bonds by C1 hydroxyl groups involved in bonding between monosaccharides was taken into consideration.

An example of determining the interaction between substituents of hydroxyl groups of a local structure in question and hydroxyl groups serving as acceptors of other monosaccharides bonded with α- or β-glucoside bonds by C1 hydroxyl groups involved in bonding between monosaccharides using the aforementioned procedure is indicated below.

Polysaccharides having similar physical quantity patterns have a high probability of belonging to polysaccharide group having similar functions.

In addition, this also serves as basic data of the structural activity correlation in local structures.

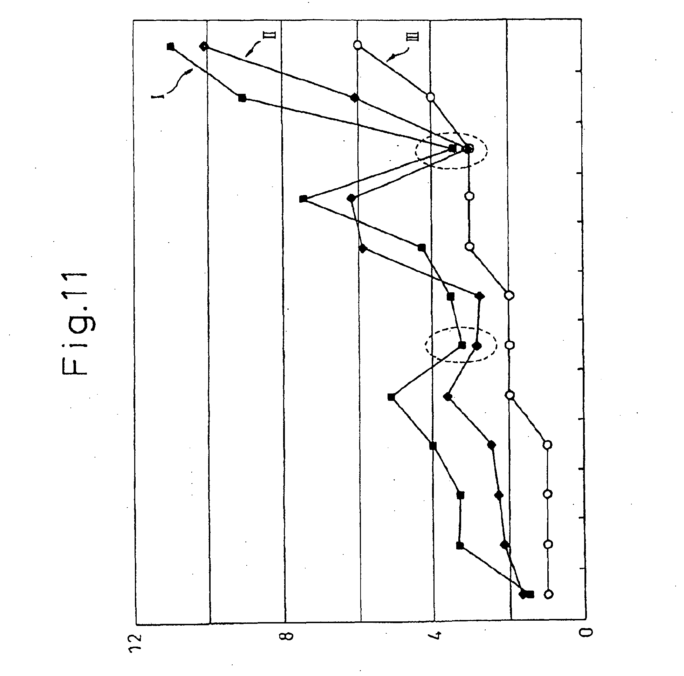

Fig. 11 shows an example of physical quantities obtained for different numbers of polysaccharides (sugar chains: SC). In the graph, plot I indicates the dipolar moment (debyes), plot II indicates the confirmed minimum energy (10 kcal/mole), and plot III indicates the SC.

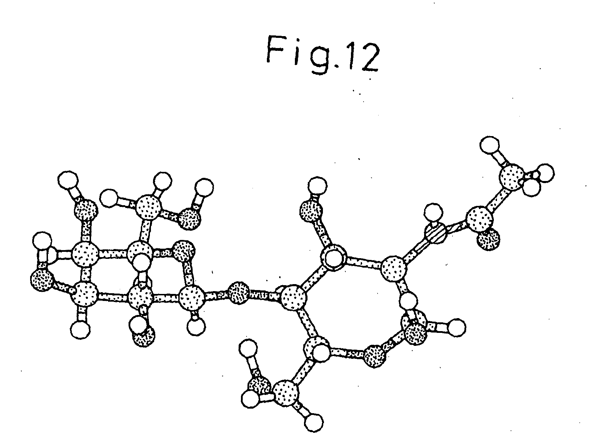

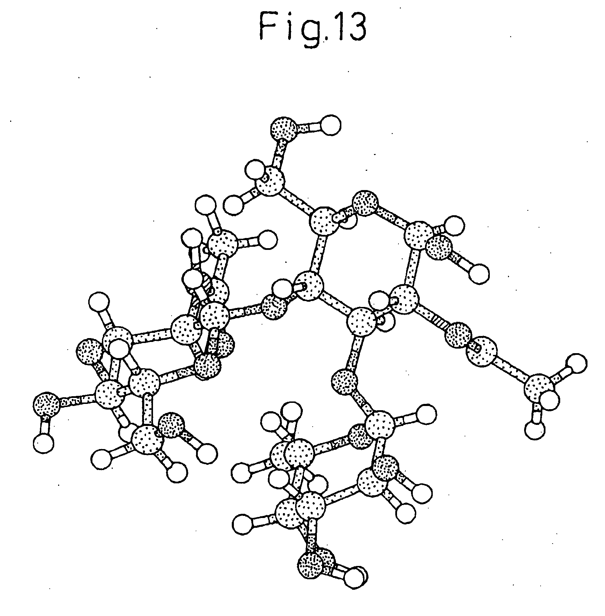

Figs. 12 and 13 show examples of outputs according to an embodiment of the present invention. Fig. 12 indicates 1LTE (legume, Dico), while Fig. 13 indicates 1LED (human blood Lewis B, cancer, adhesion). In these examples, examples of their local structures are output in the case interaction patterns with the environment that appear in all ten local structures for each location of the alignment shown in Fig. 10 are similar.

Polysaccharide coordinate data may be arbitrary data given by a user. Which properties of monosaccharides are considered and the number of groups defined are arbitrary. This monosaccharide classification information may also be input dynamically from input device 20 shown in Fig. 8.

In addition to a method and device for analyzing the three-dimensional structure of polysaccharides as has been previously described, the present invention also provides a polysaccharide three-dimensional structure analysis program used in combination with a computer when analyzing the three-dimensional structure of polysaccharides composed of monosaccharide linkages, for executing in that computer the following steps or functions:
a step in which the alignment of the local structures of a polysaccharide is input;
a step in which coordinate data relating to polysaccharides to which each local structure belongs is input;
a step in which the interactions between substituents of each hydroxyl group within a local structure and hydroxyl groups that serve as acceptors of other monosaccharides bonded by C1 hydroxyl groups involved in bonding between monosaccharides are detected;
a step in which the interactions between hydroxyl group substituents and hydroxyl groups that serve as acceptors of other monosaccharides bonded with α- or β-glucoside bonds by C1 hydroxyl groups involved in bonding between monosaccharides are evaluated based on a formal classification of bonding relating to polysaccharides; and
a step in which information is output relating to interactions with the environment that have a pattern which has been judged to be appearing in a local structure among each of the interaction patterns with the environment for each location of an alignment.

The present invention also provides a recording medium used in combination with a computer when analyzing the three-dimensional structure of polysaccharides composed of monosaccharide linkages which contains the polysaccharide three-dimensional structure analysis program of the present invention, and which can be read by a computer.

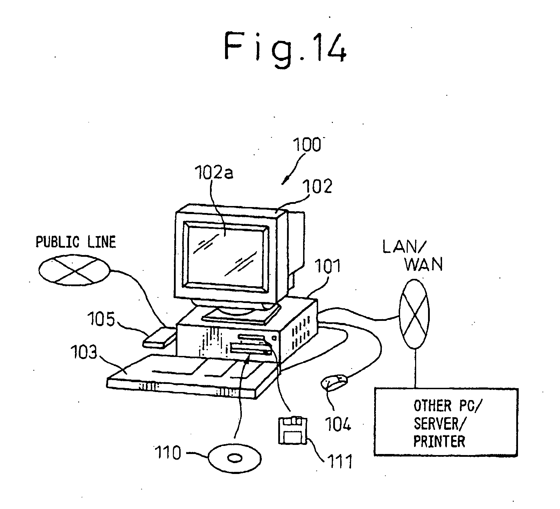

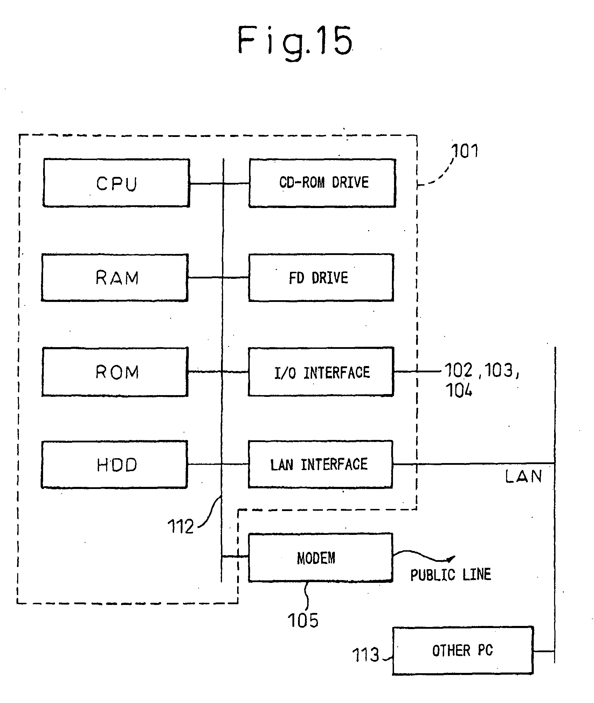

Figs. 14 and 15 are drawings for providing detailed explanations of these inventions. Fig. 14 is a perspective view schematically showing a method and device for installing a program used in a polysaccharide three-dimensional structure analysis method and device of the present invention, along with its recording medium, in a monitor, while Fig. 15 is a block diagram of the inside of the monitor shown in Fig. 14.

Monitor (hereinafter, referred to as a computer system) 100 shown in Fig. 14 is provided with a display 102 that displays image and other information on display screen 102a according to commands from a main unit 101, a keyboard 103 for inputting various information to computer system 100, a pointer (mouse) 104 that specifies an arbitrary location on display screen 102a of display 102, and a modem 105 connected to a public line.

As shown in Fig. 15, main unit 101 contains a CPU, RAM, ROM, hard disk drive (HDD), CD-ROM drive, FD drive, I/O interfaces connected to display 102, keyboard 103 and mouse 104, and an LAN interface for accessing a database connected with a public line, and these devices are interconnected by means of a bus 112.

A program of the present invention contained in a portable recording medium such as a CD-ROM 110, floppy™ disk (FD) 111, DVD disk, magnetooptical disk or IC card not shown, or in a database connected through a communication line using model 105 or an LAN interface, is installed in computer system 100 and executed with computer system 100. The installed program of the present invention is contained on a hard disk (HD) of the HDD, and is executed by the CPU using RAM and so forth.

Here, a recording medium that contains a program according to the present invention includes a portable recording medium such as CD-ROM 110, FD 111, DVD disk, magnetooptical disk or IC card, a storage device such as a hard disk drive provided within computer system 100, a database that retains the installation source of the program of the present invention and is connected by means of a communication line, an example of which is another computer system or server database indicated with reference symbol 113 connected to computer system 100 by means of an LAN, and a transfer medium for transfer over a communication line.

Moreover, the present invention provides in particular a diagnostic support system, or in other words, a method for supporting diagnoses of diseases and so forth using the method and device for analyzing the three-dimensional structures of polysaccharides according to the present invention as previously described, comprising:
a step in which polysaccharide information is input;
a step in which patient symptoms are input;
a step in which patient existing information is input;
a step in which the features possessed by polysaccharide three-dimensional structures are extracted;
a step in which pathology is evaluated based on polysaccharide information;
a step in which suitable treatment is evaluated;
a step in which suitable prevention is evaluated;
a step in which information relating to determination of pathology is output;
a step in which information relating to suitable treatment is output; and
a step in which information relating to suitable prevention is output. Furthermore, although polysaccharide information to be input can preferably be determined from the method and device for analyzing three-dimensional structures of polysaccharides according to the present invention when carrying out this diagnostic support method, this information may also be determined with other methods or devices as desired.

The use of a diagnostic support method of the present invention makes it possible to carry out examinations and treatment at hospitals and clinics easily, rapidly and appropriately. In addition, it is also able to eliminate overhead associated with diagnosis. In actuality, accurate determination of pathology, suitable treatment and suitable prevention can be carried out by physicians based on the resulting output data.

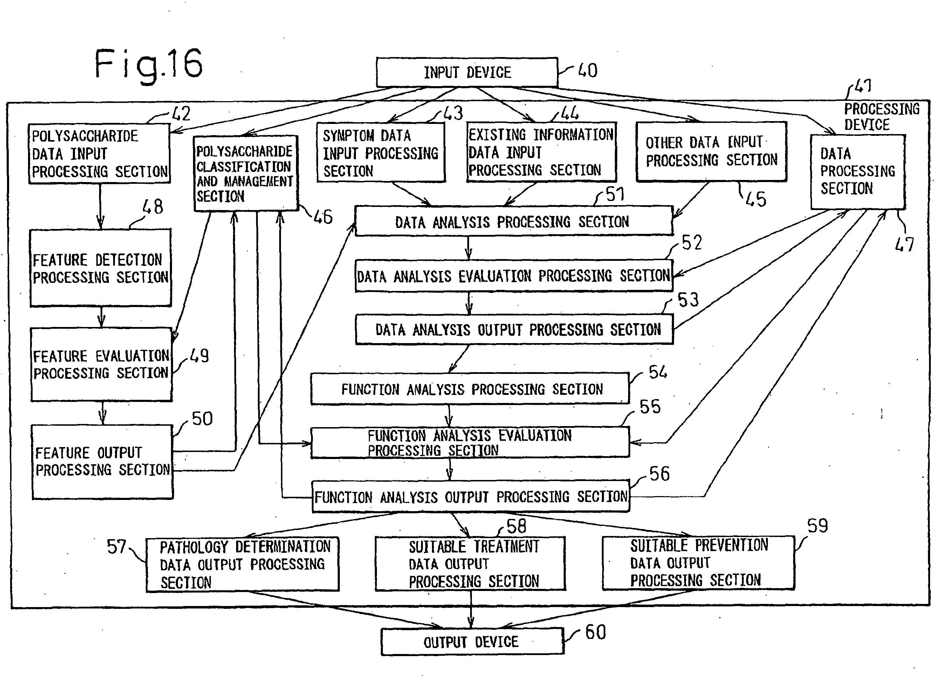

Fig. 16 is a flow chart explaining the principle of a diagnostic support method according to the present invention. As shown in Fig. 16, polysaccharide information can be determined by a method and device for analyzing the three-dimensional structure of polysaccharides according to the present invention as previously described.

The following provides a brief explanation of the principle of this diagnostic support method of the present invention with reference to Fig. 16. Data input device 40 is a device that inputs data to a data processing device 41, and includes, for example, an external storage device and a keyboard device. Data processing device 41 is provided with a CPU, memory and so forth, and executes a program that analyzes the three-dimensional structure of polysaccharides composed of monosaccharide linkages.

In data processing apparatus 41, polysaccharide data acquired with a method and device of the present invention is input to a polysaccharide data input processing section 42 using data input device 40. In addition, data input device 40 is composed to also allow input of necessary data to a polysaccharide classification and management section (database) 46, a symptom data input processing section 43, an existing information data input processing section 44, an other data input processing section 45 and a data management section 47, respectively.

Polysaccharide data that has been input to polysaccharide data input processing section 42 is processed therein after which it is output to a feature detection processing section 48 where it is processed therein, sequentially processed in a feature evaluation processing section 49 and a feature output processing section 50, and finally output to polysaccharide classification and management section 46 and a data analysis processing section 51.

In addition to data from feature output processing section 50, data from symptom data input processing section 43, existing information data input processing section 44 and other data input processing section 45 are also centralized in data analysis processing section 51 where the required data analysis is carried out.

Following completion of data analysis in data analysis processing section 51, the analysis results are output to a data analysis evaluation processing section 52 where it is processed together with data from data management section 47. Next, data from data analysis evaluation processing section 52 is output to a data analysis output processing section 53, and after the required processing is carried out therein, the data is output to a function analysis processing section 54 in order to analyze function. In addition, the data of data analysis output processing section 53 is also fed back to data management section 47 where it is stored as shown in the drawing.

As illustrated, the function analysis process is carried out by functional analysis processing section 54, function analysis evaluation processing section 55 and function analysis output processing section 56 in that order. As a result, pathology determination data, suitable treatment data and suitable prevention data are output from function analysis output processing section 56. Furthermore, although only these three types of data are shown in the drawing for the sake of convenience, other data can also be output as necessary. In addition, the data of function analysis output processing section 56 is also fed back to polysaccharide classification and management section 64 and data management section 47.

The data of function analysis output processing section 56 is input to output device 60 via pathology determination data output processing section 57, suitable treatment data output processing section 58 and suitable prevention data output processing section 59, respectively. Accordingly, pathology determination data, suitable treatment data and suitable prevention data and so forth can be output by output device 60 for use by physicians by going through the aforementioned series of processing steps.

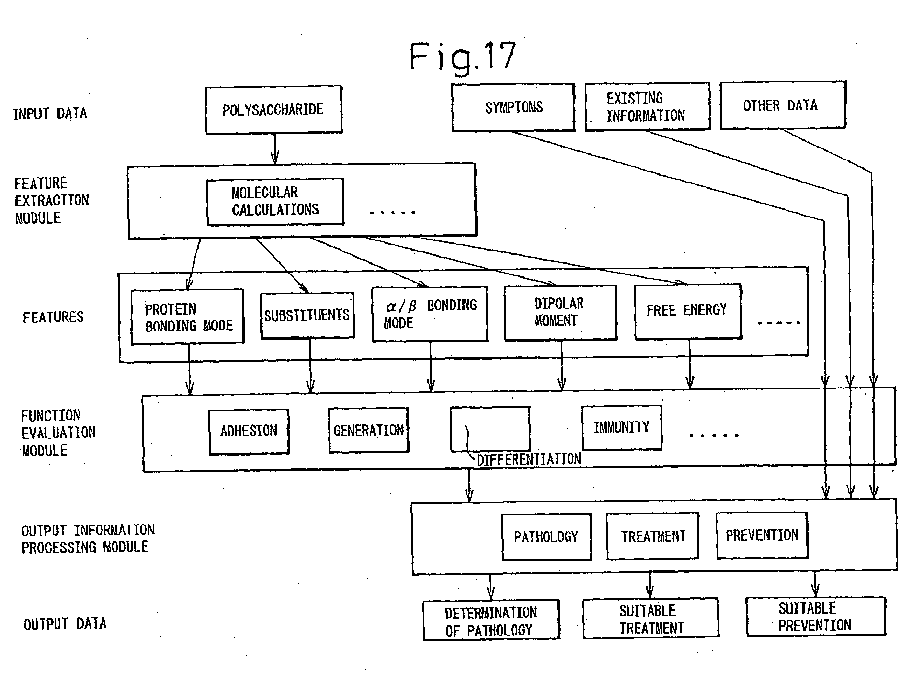

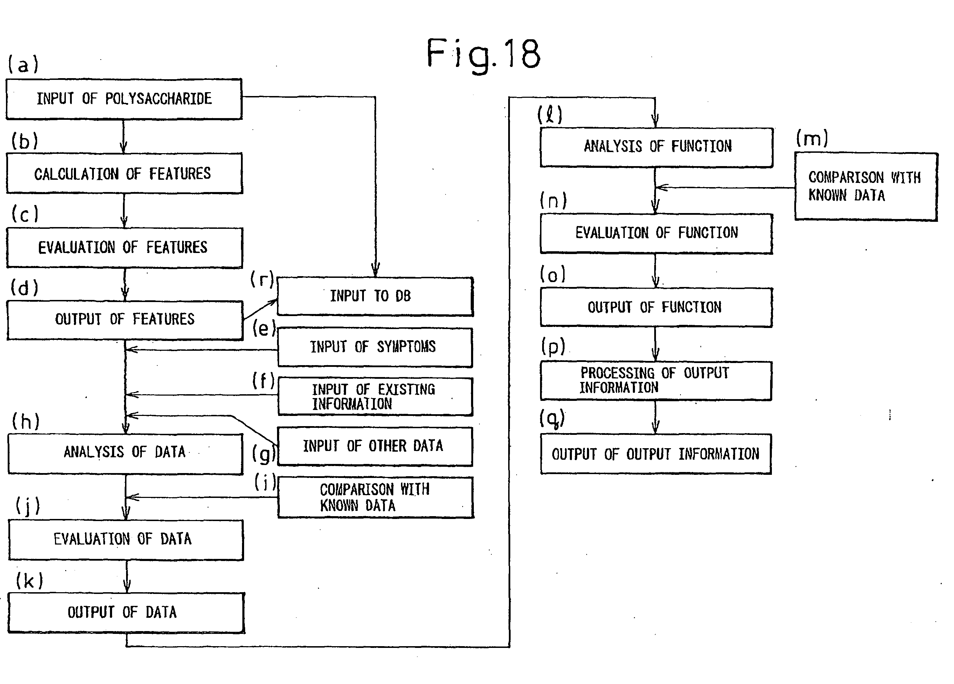

In addition, a preferred embodiment of a diagnostic support method according to the present invention is shown in Fig. 17, and its processing flow is shown in Fig. 18 for reference purposes. Furthermore, the molecular calculations shown in Fig. 17 can be carried out by MO, MD, MM, QM and so forth.

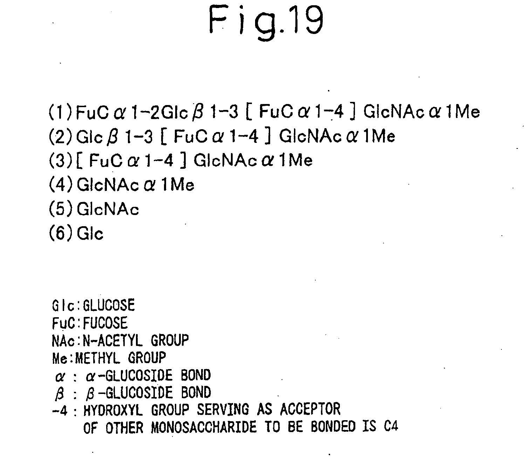

Fig. 19 shows an example of a polysaccharide composition that can be input by a polysaccharide data input processing section in the practice of the present invention. Furthermore, this example of polysaccharide data that can be input by a polysaccharide data input processing section in the practice of the present invention can be the same as that shown in the previously explained Fig. 5.

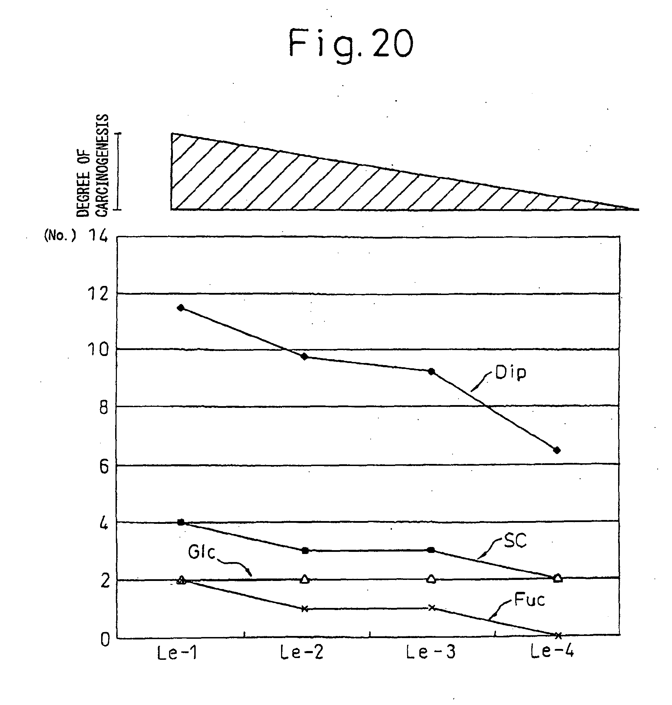

In addition, Fig. 20 shows an example of an output according to an example of the present invention. In this drawing, a tendency towards carcinogenesis (degree of carcinogenesis) is observed from the numbers for DiP (dipolar moment), SC (sugar chain), Glc (glucose) and Fuc (fucose). As can be understood from Fig. 20, use of the diagnostic support method of the present invention makes it possible to accurately observe tendencies toward carcinogenesis.

### INDUSTRIAL APPLICABILITY

As has been explained above, according to the present invention, interaction patterns between substituents of hydroxyl groups and hydroxyl groups serving as acceptors of other monosaccharides bonded with α- or β-glucoside bonds by C1 hydroxyl groups involved in bonding between monosaccharides, not only within local structures but also of hydroxyl groups that form the environment surrounding local structures, can be characterized when characterizing monosaccharide bonding patterns common to a plurality of local structures.

In addition, since results are output automatically simply by inputting alignments and coordinate data without being mediated by bothersome work or being susceptible to the introduction of subjectivity involving detailed examination of three-dimensional diagrams and so forth, a large amount of data can be processed and even in the case of trial-and-error analyses requiring alteration of parameters, analyses can be carried out systematically in accordance with a predetermined procedure. Thus, the present invention greatly contributes to the development of three-dimensional structure prediction methods as a result of being useful for the construction of empirical rules for correlating monosaccharide bonding with polysaccharide three-dimensional structure.

Moreover, although the present invention can be widely used in various fields involving the use of polysaccharides, it can be used particularly effectively in the fields of tailor-made drug development and pathological diagnosis.

In addition, according to the present invention, changes in protein structures caused by abnormalities in homeostasis can be extracted by detecting features such as physical quantities possessed by the three-dimensional structure of polysaccharides. In addition, since results are output automatically simply by inputting data without being mediated by bothersome work or being susceptible to the introduction of subjectivity involving detailed examination of three- dimensional diagrams and so forth, a large amount of data can be processed and even in the case of trial-and-error analyses requiring alteration of parameters, analyses can be carried out systematically in accordance with a predetermined procedure. Thus, the present invention greatly contributes to the development of diagnostic support methods by being useful for the construction of empirical rules for correlating monosaccharide bonding with polysaccharide three-dimensional structure.

## Claims

1. A method for analyzing the three-dimensional structure of polysaccharides composed of monosaccharide linkages, comprising:
inputting the alignment of the local structures of a polysaccharide;
inputting coordinate data relating to polysaccharides to which each local structure belongs;
detecting the interactions between substituents of each hydroxyl group within a local structure and hydroxyl groups that serve as acceptors of other monosaccharides bonded by C1 hydroxyl groups involved in bonding between monosaccharides;
evaluating the interactions between hydroxyl group substituents and hydroxyl groups that serve as acceptors of other monosaccharides bonded with α- or β-glucoside bonds by C1 hydroxyl groups involved in bonding between monosaccharides, based on a formal classification of bonding relating to polysaccharides; and
outputting information relating to interactions with the environment that have a pattern which has been judged to be appearing in a local structure among each of the interaction patterns with the environment for each location of an alignment.

2. A method according to claim 1 wherein the polysaccharide is a high-mannose type, mannose-6-phosphate type, complex type, single-chain complex type, double-chain complex type, triple-chain complex type, quadruple-chain complex type, diallyl Lex type or bisecting complex type polysaccharide.

3. A method according to claim 1 or 2 wherein the interactions are represented by dipolar moment calculated from the molecular orbit method, free energy calculated from the molecular dynamics method, dipolar moment for hydrophobicity or electrostatic potential for hydrophilicity calculated from the molecular orbit method, or the properties of frontier orbitals.

4. A device for analyzing the three-dimensional structure of polysaccharides composed of monosaccharide linkages, comprising a data input device that inputs data, a data processing device that executes commands, a data management device that manages data, and a data output device that outputs data; wherein the data processing device comprises:
an alignment data input processing unit that inputs the alignments of polysaccharide local structures;
a coordinate data input processing unit that inputs coordinate data relating to polysaccharides to which each local structure belongs;
an interaction detection processing unit that detects an interaction between substituents of each hydroxyl group in a local structure and hydroxyl groups serving as acceptors of other monosaccharides bonded by C1 hydroxyl groups involved in bonding between monosaccharides;
an interaction pattern evaluation processing unit that evaluates patterns of interaction between hydroxyl group substituents and hydroxyl groups serving as acceptors of other monosaccharides bonded with α- or β-glucoside bonds by C1 hydroxyl groups involved in bonding between monosaccharides based on a formal classification of bonding relating to polysaccharides; and
an interaction pattern output processing unit that outputs information relating to interactions with the environment that have a pattern which has been judged to be appearing in a local structure among each of the interaction patterns with the environment for each location of an alignment.

5. A device according to claim 4 wherein the polysaccharide is a high-mannose type, mannose-6-phosphate type, complex type, single-chain complex type, double-chain complex type, triple-chain complex type, quadruple-chain complex type, diallyl Lex type or bisecting complex type polysaccharide.

6. A device according to claim 4 or 5 wherein the interactions are represented by dipolar moment calculated from the molecular orbit method, free energy calculated from the molecular dynamics method, dipolar moment for hydrophobicity or electrostatic potential for hydrophilicity calculated from the molecular orbit method, or the properties of frontier orbitals.

7. A polysaccharide three-dimensional structure analysis program used in combination with a computer when analyzing the three-dimensional structure of polysaccharides composed of monosaccharide linkages, for executing in said computer the following:
inputting the alignment of the local structures of a polysaccharide;
inputting coordinate data relating to polysaccharides to which each local structure belongs;
detecting the interactions between substituents of each hydroxyl group within a local structure and hydroxyl groups that serve as acceptors of other monosaccharides bonded by C1 hydroxyl groups involved in bonding between monosaccharides;
evaluating the interactions between hydroxyl group substituents and hydroxyl groups that serve as acceptors of other monosaccharides bonded with α- or β-glucoside bonds by C1 hydroxyl groups involved in bonding between monosaccharides, based on a formal classification of bonding relating to polysaccharides; and
outputting information relating to interactions with the environment that have a pattern which has been judged to be appearing in a local structure among each of the interaction patterns with the environment for each location of an alignment.

8. A program according to claim 7 wherein the polysaccharide is a high-mannose type, mannose-6-phosphate type, complex type, single-chain complex type, double-chain complex type, triple-chain complex type, quadruple-chain complex type, diallyl Lex type or bisecting complex type polysaccharide.

9. A program according to claim 7 or 8 wherein the interactions are represented by dipolar moment calculated from the molecular orbit method, free energy calculated from the molecular dynamics method, dipolar moment for hydrophobicity or electrostatic.potential for hydrophilicity calculated from the molecular orbit method, or the properties of frontier orbitals.

10. A recording medium used in combination with a computer when analyzing the three-dimensional structure of polysaccharides composed of monosaccharide linkages which contains a polysaccharide three-dimensional structure analysis program according to any one of claims 7 to 9, and which can be read by a computer.

11. A method for analyzing the three-dimensional structure of polysaccharides composed of monosaccharide linkages comprising:
inputting the alignment of the local structures of a polysaccharide;
inputting data on characteristic interaction patterns with the environment, features and so forth relating to polysaccharides to which each local structure belongs;
detecting the interactions between substituents of each hydroxyl group within a local structure and hydroxyl groups that serve as acceptors of other monosaccharides bonded by C1 hydroxyl groups involved in bonding between monosaccharides;
evaluating the interactions between hydroxyl group substituents and hydroxyl groups that serve as acceptors of other monosaccharides bonded with α- or β-glucoside bonds by C1 hydroxyl groups involved in bonding between monosaccharides, based on a formal classification of bonding relating to polysaccharides; and
outputting information relating to interactions with the environment that have a pattern which has been judged to be appearing in a local structure among each of the interaction patterns with the environment for each location of an alignment.

12. A method according to claim 11 which, in combination with the step in which data on characteristic interaction patterns with the environment, features and so forth relating to polysaccharides to which each local structure belongs is input, additionally comprises:
classifying and managing polysaccharide data;
analysising and processing data to detect polysaccharides having similar interaction patterns with respect to the resulting interaction patterns;
analysising, evaluating and processing data to evaluate polysaccharides having similar interaction patterns, based on a formal classification of bonding relating to polysaccharides; and
analysising data and output processing to output information in which polysaccharides have been judged to be similar in each of the interaction patterns.

13. A device for analyzing the three-dimensional structure of polysaccharides composed of monosaccharide linkages which comprises a data input device that inputs data, a data processing device that executes commands, a data management device that manages data, and a data output device that outputs data; wherein the data processing device comprises:
an alignment data input processing unit that inputs the alignments of polysaccharide local structures;
a coordinate data input processing unit that inputs coordinate data relating to polysaccharides to which each local structure belongs;
another data input processing unit that inputs data on characteristic interaction patterns with the environment, features and so forth relating to polysaccharides to which each local structure belongs;
an interaction detection processing unit that detects an interaction between substituents of each hydroxyl group in a local structure and hydroxyl groups serving as acceptors of other monosaccharides bonded by C1 hydroxyl groups involved in bonding between monosaccharides;
an interaction pattern evaluation processing unit that evaluates patterns of interaction between hydroxyl group substituents and hydroxyl groups serving as acceptors of other monosaccharides bonded with α- or β-glucoside bonds by C1 hydroxyl groups involved in bonding between monosaccharides based on a formal classification of bonding relating to polysaccharides; and
an interaction pattern output processing unit that outputs information relating to interactions with the environment that have a pattern which has been judged to be appearing in a local structure among each of the interaction patterns with the environment for each location of an alignment.

14. A device according to claim 13 which, in combination with the other data input processing unit that inputs data on characteristic interaction patterns with the environment, features and so forth relating to polysaccharides to which each local structure belongs, additionally comprises:
a polysaccharide classification and management unit that classifies and manages polysaccharide data;
a data analysis processing unit that detects polysaccharides having similar interaction patterns with respect to the resulting interaction patterns;
a data analysis evaluation processing unit that evaluates polysaccharides having similar interaction patterns based on a formal classification of bonding relating to polysaccharides; and
a data analysis output processing unit that outputs information in which polysaccharides have been judged to be similar in each of the interaction patterns.

15. A polysaccharide three-dimensional structure analysis program used in combination with a computer when analyzing the three-dimensional structure of polysaccharides composed of monosaccharide linkages, for executing in said computer the following:
inputting the alignment of the local structures of a polysaccharide;
inputting data on characteristic interaction patterns with the environment, features and so forth relating to polysaccharides to which each local structure belongs;
detecting the interactions between substituents of each hydroxyl group within a local structure and hydroxyl groups that serve as acceptors of other monosaccharides bonded by C1 hydroxyl groups involved in bonding between monosaccharides;
evaluating the interaction patterns between hydroxyl group substituents and hydroxyl groups that serve as acceptors of other monosaccharides bonded with α- or β-glucoside bonds by C1 hydroxyl groups involved in bonding between monosaccharides, based on a formal classification of bonding relating to polysaccharides; and
outputting information relating to interactions with the environment that have a pattern which has been judged to be appearing in a local structure among each of the interaction patterns with the environment for each location of an alignment.

16. A polysaccharide three-dimensional structure analysis program according to claim 15 which, in combination with the step in which data on characteristic interaction patterns with the environment, features and so forth relating to polysaccharides to which each local structure belongs is input, additionally comprises:
classifying and managing polysaccharide data;
data analysis processing to detect polysaccharides having similar interaction patterns with respect to the resulting interaction patterns;
data analysis evaluation processing to evaluate in which polysaccharides having similar interaction patterns, based on a formal classification of bonding relating to polysaccharides; and
data analysis output processing to output information in which polysaccharides have been judged to be similar in each of the interaction patterns.

17. A recording medium used in combination with a computer when analyzing the three-dimensional structure of polysaccharides composed of monosaccharide linkages which contains a polysaccharide three-dimensional structure analysis program according to claim 15 or 16, and which can be read by a computer.

18. A method for supporting diagnoses of diseases and so forth using the three-dimensional structures of polysaccharides composed of monosaccharide linkages, comprising:
inputting polysaccharide information;
inputting patient symptoms;
inputting patient existing information;
extracting the features possessed by polysaccharide three-dimensional structures;
evaluating pathology based on polysaccharide information;
evaluating suitable treatment;
evaluating suitable prevention;
outputting information relating to determination of pathology;
outputting information relating to suitable treatment is output; and
outputting information relating to suitable prevention.

19. A method for supporting diagnoses of diseases and so forth using the three-dimensional structures of polysaccharides composed of monosaccharide linkages, comprising:
evaluating pathology based on polysaccharide information;
evaluating suitable treatment;
evaluating suitable prevention;
outputting information relating to determination of pathology;
outputting information relating to suitable treatment; and
outputting information relating to suitable prevention.
